# EUROPEAN PATENT APPLICATION

(11) **EP 4 585 205 A1**
(43) Date of publication of application: **16.07.2025**
(21) Application number: 23863100.6
(22) Date of filing: 31.08.2023
(51) Int. Cl.: A61K 8/86, A61K 8/39, A61Q 1/00, A61Q 5/00, A61Q 19/00

(54) **COSMETIC BASE**

(30) Priority: 05.09.2022 JP 2022140912
(71) Applicant: Shiseido Company, Ltd., Chuo-ku Tokyo 104-0061 (JP); NOF Corporation, Tokyo 150-6012 (JP)
(72) Inventor: ITO, Yuji, Tokyo 104-0061 (JP); ISHIKAWA, Riko, Tokyo 104-0061 (JP); IETANI, Saori, Tokyo 104-0061 (JP); HARA, Yusuke, Kawasaki-shi, Kanagawa 210-0865 (JP); MURAI, Masaki, Kawasaki-shi, Kanagawa 210-0865 (JP); SEKIGUCHI, Koji, Kawasaki-shi, Kanagawa 210-0865 (JP)
(74) Representative: Ter Meer Steinmeister & Partner
(86) International application number: PCT/JP2023/031885
(87) International publication number: WO 2024/053562

(57) **Abstract**

Provided is a novel cosmetic base that achieves both a use sensation and a moisturizing effect. The cosmetic base contains or consists of a polymer having the structure represented by formula (1) wherein, in formula (1), R¹ is a C1-4 alkyl group, R² and R³ are each independently a hydrogen atom or C1-4 alkyl group, m and n are each independently 1 to 50, the number-average molecular weight of the polymer is not greater than 10,000, and the IOB value of the polymer is 0.4 to 1.8.

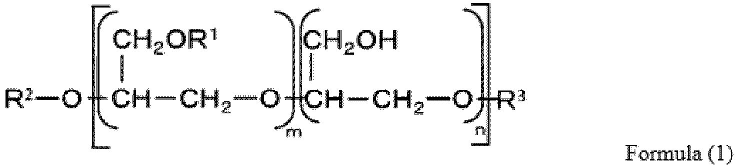

## Description

### FIELD

The present invention relates to a cosmetic base.

### BACKGROUND

To maintain healthy skin, retention of moisture is essential, and a large number of cosmetics have been developed for the purpose of retaining moisture. In terms of feeling of use of cosmetics, those having a moisturized feeling without any sticky feeling have been in demand.

Various cosmetics or cosmetic bases have been developed so far for the purpose of moisturizing.

PTL 1, for example, discloses a moisturizer containing a polyether represented by general formula (1):

-[X]ₘ-[Y]ₙ- (1)

wherein m number of X each represent a group (X¹) or (X²); n number of Y each represent a group derived from a monomer copolymerizable with an epoxide (x¹) or (x²); m and n each represent a number that satisfies m/(m + n) = 0.2 to 1 and m + n ≥ 12, 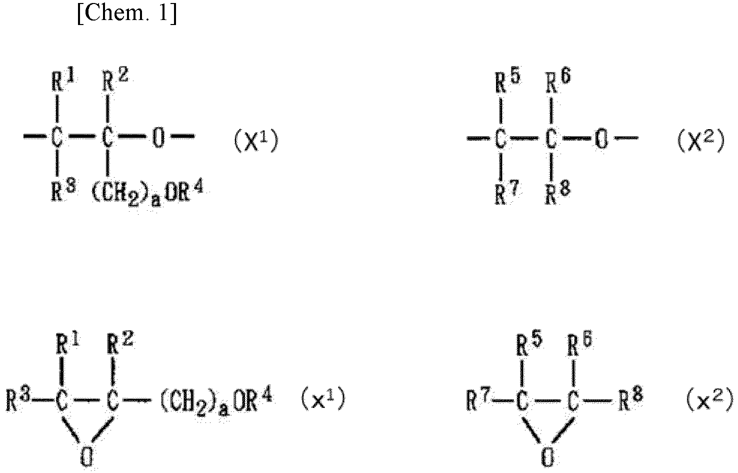
where R¹ to R³ is H, Me, or Et; R⁴ is H or a hydrocarbon group having C1 to C32; a is 1 or 2; and R⁵ to R⁸ are H, -COOH, -CH₂COOH, or a salt thereof, excluding R⁵ = R⁶ = R⁷ = R⁸ = H.

PTL 2 discloses a topical skin preparation comprising an alkylene oxide derivative represented by general formula (I) below:
[Chem. 2]

R¹O-[(AO)ₘ(EO)ₙ]-R² (I)

wherein AO is an oxyalkylene group having 3 or 4 carbon atoms; EO is an oxyethylene group; m and n are average numbers of moles of the oxyalkylene group and the oxyethylene group added, respectively, where 1 ≤ m ≤ 70 and 1 ≤ n ≤ 70; a ratio of oxyethylene groups relative to a total of oxyalkylene groups having 3 or 4 carbon atoms and oxyethylene groups is 20 to 80% by weight; the oxyalkylene groups having 3 or 4 carbon atoms and the oxyethylene groups may be added in a block or random manner; R¹ and R² are each a hydrocarbon group having 1 to 4 carbon atoms or a hydrogen atom, which may be the same or different; and a ratio of the number of hydrogen atoms relative to the number of hydrocarbon groups among R¹ and R² is 0.15 or less.

### [CITATION LIST]

### [PATENT LITERATURE]

[PTL 1] Japanese Unexamined Patent Publication (Kokai) No. 2001-048772
[PTL 2] Japanese Unexamined Patent Publication (Kokai) No. 2004-083541

### SUMMARY

### [TECHNICAL PROBLEM]

For example, the moisturizer that has been disclosed in PTL 1 mentioned above may not be satisfactory in terms of feeling of use as a cosmetic, due to the structure (for example, type of substituent and molecular weight) of the polyether represented by the general formula (1) (-[X]ₘ-[Y]n-).

The present invention aims to improve the above circumstances, and an object thereof is to provide a new cosmetic base that achieves a balance of feeling of use and a moisturizing effect.

### [SOLUTION TO PROBLEM]

The present invention that achieves the above object is as follows.

### <Aspect 1>

A cosmetic base, comprising or composed of a polymer having a structure represented by Formula (1) below: 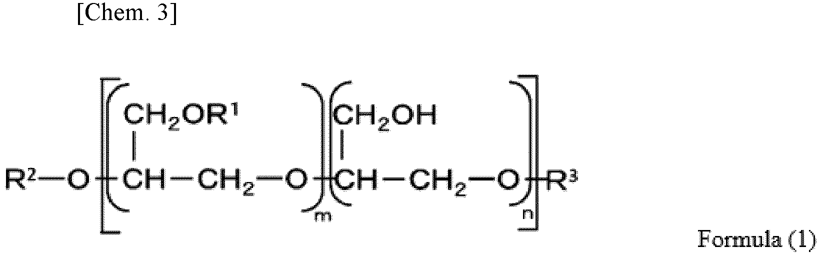
wherein in the Formula (1), R¹ is an alkyl group having 1 to 4 carbon atoms; R² and R³ are each independently a hydrogen atom or an alkyl group having 1 to 4 carbon atoms; and m and n are each independently 1 to 50,
wherein a number average molecular weight of the polymer is 10,000 or less, and
wherein an IOB value of the polymer is 0.4 to 1.8.

### <Aspect 2>

The cosmetic base according to Aspect 1, wherein the polymer is a random copolymer.

### <Aspect 3>

The cosmetic base according to Aspect 1 or 2, wherein in the Formula (1), R¹ is a methyl group or an ethyl group.

### <Aspect 4>

The cosmetic base according to any one of Aspects 1 to 3, wherein in the Formula (1), at least one of R² and R³ is a methyl group.

### <Aspect 5>

The cosmetic base according to any one of Aspects 1 to 4, which is used for moisturizing.

### <Aspect 6>

A cosmetic, comprising the cosmetic base according to any one of Aspects 1 to 5.

### [ADVANTAGEOUS EFFECTS OF INVENTION]

According to the present invention, a new cosmetic base that achieves a balance of feeling of use and a moisturizing effect can be provided.

### DESCRIPTION OF EMBODIMENTS

Hereinafter, embodiments of the present invention will be described in detail. Note that, the present invention is not limited to the following embodiments, and various modifications can be made within the scope of the invention.

### <<Cosmetic base>>

The cosmetic base of the present invention
comprises or is composed of a polymer having a structure represented by Formula (1) below: 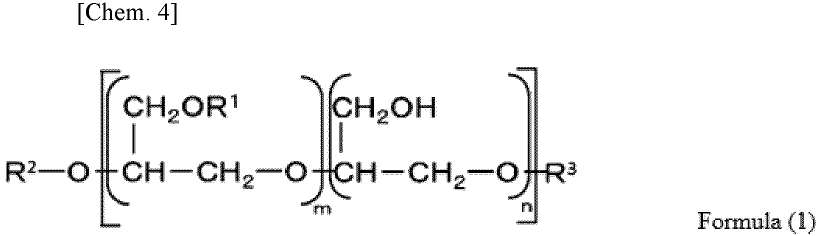
wherein in Formula (1), R¹ is an alkyl group having 1 to 4 carbon atoms; R² and R³ are each independently a hydrogen atom or an alkyl group having 1 to 4 carbon atoms; and m and n are each independently 1 to 50,
wherein a number average molecular weight of the polymer is 10,000 or less, and
wherein an IOB value of the polymer is 0.4 to 1.8.

The present inventors, through intensive research, have discovered that the cosmetic base of the present invention can achieve a balance of feeling of use and a moisturizing effect by comprising a polymer having the structure represented by Formula (1) mentioned above (hereinafter, also called "polymer of the present invention") or being composed of the polymer of the present invention.

Without being bound by theory, it is presumed that the above is derived from the polymer of the present invention having the unique structure shown in Formula (1) above, a number average molecular weight of a specific range (10,000 or less), and an IOB (Inorganic Organic Balance) value of a specific range (0.4 to 1.8).

The cosmetic base of the present invention can contain, as components other than the polymer of the present invention, water; an antioxidant such as tocopherol; and a preservative such as phenoxyethanol, or may consist only of the polymer of the present invention. In the cosmetic base of the present invention, when components other than the polymer of the present invention is contained, the content thereof may be 50% by mass or less, 40% by mass or less, 30% by mass or less, 20% by mass or less, 10% by mass or less, 5% by mass or less, or 1% by mass or less.

The feeling of use from the cosmetic base of the present application is determined comprehensively based on "moisturized feeling", "sticky feeling", "ease of blending into and spreading on skin", and "slimy feeling".

Hereinafter, the polymer of the present invention will be described in detail. Note that, in the following description, unless otherwise specified, the term "Formula (1)" refers to chemical formulas related to the present invention, not formulas in prior art literature.

The polymer of the present invention has a structure represented by Formula (1) below:

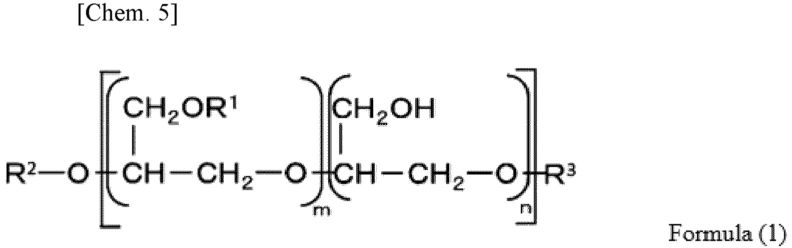

In Formula (1), R¹ is an alkyl group having 1 to 4 carbon atoms, and R² and R³ are each independently a hydrogen atom or an alkyl group having 1 to 4 carbon atoms. In the present invention, examples of the alkyl group having 1 to 4 carbon atoms include a methyl group, an ethyl group, an n-propyl group, an isopropyl group, an n-butyl group, a sec-butyl group, and a tert-butyl group. These alkyl groups may further have a substituent as long as the effect of the present invention is not impaired. The substituent is not particularly limited, and examples thereof include halogen groups.

According to one embodiment of the present invention, in Formula (1), R¹ may be a methyl group, an ethyl group, an n-propyl group, an isopropyl group, an n-butyl group, a sec-butyl group, or a tert-butyl group.

According to one embodiment of the present invention, in Formula (1), R¹ is preferably a methyl group or an ethyl group, and more preferably an ethyl group.

According to one embodiment of the present invention, in Formula (1), R² and R³ may each independently be a hydrogen atom, a methyl group, an ethyl group, an n-propyl group, an isopropyl group, an n-butyl group, a sec-butyl group, or a tert-butyl group.

According to one embodiment of the present invention, in Formula (1), R² is preferably a hydrogen atom, a methyl group, or an ethyl group, and in particular, more preferably a methyl group.

According to one embodiment of the present invention, in Formula (1), R² may be in a state where a hydrogen atom and an alkyl group having 1 to 4 carbon atoms are mixed. Specifically, one portion of R² may include a hydrogen atom, and the other portion may include an alkyl group having 1 to 4 carbon atoms. Preferably, one portion of R² includes a hydrogen atom, and the other portion includes a methyl group.

According to one embodiment of the present invention, in Formula (1), R³ is preferably a hydrogen atom, a methyl group, or an ethyl group, and in particular, more preferably a methyl group.

According to one embodiment of the present invention, in Formula (1), at least one of R² and R³ is preferably a methyl group.

In Formula (1), m and n each represent an average number of moles of a structural unit added. The m and n are each independently 1.0 to 50, and more specifically, may be 1.0 or greater, 2.0 or greater, 3.0 or greater, 4.0 or greater, 5.0 or greater, 6.0 or greater, 7.0 or greater, 8.0 or greater, 9.0 or greater, or 10 or greater, and may be 50 or less, 45 or less, 40 or less, 35 or less, 30 or less, 25 or less, 20 or less, 15 or less, 12 or less, 10 or less, 8.0 or less, or 6.0 or less.

In Formula (1), m is preferably 2.0 or greater and 15 or less, or 3.0 or greater and 10 or less. In Formula (1), n is preferably 2.0 or greater and 10 or less, or 2.0 or greater and 6.0 or less.

According to one embodiment of the present invention, in Formula (1), m + n may be 4.0 or greater, 5.0 or greater, 6.0 or greater, 7.0 or greater, 8.0 or greater, 9.0 or greater, 10 or greater, 11 or greater, 12 or greater, 13 or greater, 14 or greater, or 15 or greater, and may be 50 or less, 45 or less, 40 or less, 35 or less, 30 or less, 25 or less, 20 or less, 15 or less, or 12 or less.

According to one embodiment of the present invention, in Formula (1), n:m may be 1:10 to 10:1.

In the polymer of the present invention, the number average molecular weight is 10,000 or less. More specifically, the number average molecular weight of the polymer of the present invention, for example, may be 10,000 or less, 5,000 or less, 3,500 or less, 3,000 or less, 2,500 or less, 2,000 or less, 1,500 or less, 1,400 or less, 1,300 or less, 1,200 or less, 1,100 or less, 1,000 or less, 900 or less, 800 or less, 700 or less, or 600 or less, and may be 130 or greater, 150 or greater, 200 or greater, 250 or greater, 300 or greater, 350 or greater, 400 or greater, 450 or greater, or 500 or greater. The number average molecular weight of the polymer of the present invention is preferably 300 or greater and 3,500 or less, and more preferably 500 or greater and 1,200 or less.

The IOB value of the polymer of the present invention is 0.4 to 1.8. More specifically, the IOB value of the polymer of the present invention, for example, may be 0.4 or greater, 0.5 or greater, 0.6 or greater, 0.7 or greater, 0.8 or greater, or 0.9 or greater, and may be 1.8 or less, 1.6 or less, 1.4 or less, 1.2 or less, or 1.0 or less. The IOB value of the polymer of the present invention is preferably 0.7 or greater and 1.2 or less.

The IOB value, an abbreviation for Inorganic/Organic Balance (inorganic/organic ratio), is a value that represents a ratio of inorganic value to organic value, and is an indicator showing the degree of polarity of an organic compound. The IOB value, specifically, is represented as IOB value = inorganic value / organic value. Regarding each of the "inorganic value" and "organic value", for example, the "inorganic value" and "organic value" are set according to various atoms or functional groups, based on the "organic value" for one carbon atom in a molecule being 20 and the "inorganic value" for one hydroxyl group being 100. By integrating the "inorganic value" and "organic value" of all atoms and functional groups in an organic compound, the IOB value of the organic compound can be calculated (refer to, for example, Yoshio Koda, "Organic Concept Map - Basics and Applications -", pp. 11 to 17, Sankyo Publishing Co., Ltd., 1984). Regarding a method for determining the IOB value, the method described in the Examples can be referenced.

The polymer of the present invention may be a block copolymer, or may be a random copolymer, but is preferably a random copolymer.

The method of manufacturing the polymer of the present invention is not particularly limited, and for example, may be carried out by addition polymerization of epoxides corresponding to each structural unit. The addition polymerization may be block polymerization, or may be random polymerization, but is preferably random polymerization.

The polymer of the present invention, as one of the characteristics thereof, has both high water solubility and high lipid solubility.

The degree of solubility in water of the polymer of the present invention, for example, can be 10% by mass or greater, 20% by mass or greater, 30% by mass or greater, 40% by mass or greater, or 50% by mass or greater.

As an indicator of lipid solubility of the polymer of the present invention, for example, degree of solubility in olive oil can be used. The degree of solubility in olive oil of the polymer of the present invention, for example, can be 0.01% by mass or greater, 0.05% by mass or greater, 0.10% by mass or greater, 0.50% by mass or greater, or 1.00% by mass or greater. Note that, the upper limit value of the degree of solubility in olive oil of the polymer of the present invention is not particularly limited, and for example, may be 10% by mass or less.

The polymer of the present invention, according to the above characteristics, can be used, for example, as a substitute of the alkylene oxide derivative of PTL 2. Specifically, a cosmetic base comprising the polymer of the present invention may be used for moisturizing.

### <<Cosmetic>>

The present invention also provides a cosmetic comprising the cosmetic base of the present invention.

The content of the cosmetic base of the present invention mentioned above in the cosmetic of the present invention is not particularly limited. For example, the polymer of the present invention in the entirety of the cosmetic may be in an amount of 0.001 to 90% by mass, 0.1 to 50% by mass, or 0.1 to 20% by mass, or further 0.1 to 10% by mass. More specifically, for example, the polymer of the present invention relative to the entirety of the cosmetic may be 0.001% by mass or greater, 0.005% by mass or greater, 0.01% by mass or greater, 0.05% by mass or greater, 0.1% by mass or greater, 0.5% by mass or greater, 1.0% by mass or greater, 1.5% by mass or greater, or 2.0% by mass or greater, and may be 90% by mass or less, 80% by mass or less, 70% by mass or less, 60% by mass or less, 50% by mass or less, 40% by mass or less, 30% by mass or less, 20% by mass or less, 10% by mass or less, 9.0% by mass or less, 8.0% by mass or less, 7.0% by mass or less, 6.0% by mass or less, 5.0% by mass or less, 4.0% by mass or less, 3.0% by mass or less, 2.0% by mass or less, or 1.0% by mass or less.

The cosmetic of the present invention, in addition to the cosmetic base of the present invention mentioned above, may further comprise, as additional components, one or more components used in topical skin preparations such as ordinary cosmetics and pharmaceuticals.

Specifically, examples of the additional components include powder components, liquid oils and fats, solid oils and fats, waxes, hydrocarbons, higher fatty acids, higher alcohols, esters, silicones, anionic surfactants, cationic surfactants, amphoteric surfactants, nonionic surfactants, water-soluble polymers, thickeners, film-forming agents, UV absorbers, sequestering agents, lower alcohols, polyhydric alcohols, saccharides, amino acids, organic amines, polymer emulsions, pH modifiers, skin nutrients, vitamins, antioxidants, antioxidant aids, perfumes, and water, but are not limited thereto.

The formulation of the cosmetic of the present invention is not particularly limited, and for example, may be a solution system, a solubilized system, an emulsion system, a powder dispersion system, a water-oil two-layer system, a water-oil-powder three-layer system, a gel, a mist, a spray, a mousse, a roll-on, or a stick, or may be a preparation impregnated or applied to a sheet such as a nonwoven fabric.

The product form of the cosmetic of the present invention is not particularly limited, and for example, may be a facial cosmetic such as a toner, a milky lotion, a cream, or a pack; a make-up cosmetic such as a foundation, a lipstick, or an eye shadow; a sunscreen cosmetic (sunscreen agent); a body cosmetic; a fragranced cosmetic; a skin cleanser such as a make-up remover or a body shampoo; a hair cosmetic such as a hair liquid, a hair tonic, a hair conditioner, a shampoo, a rinse, or a hair growth treatment; or an ointment.

### EXAMPLES

The present invention will be further described in detail with reference to the Examples below. However, the present invention is not limited thereto.

### <<Synthesis Examples 1 and 2>>

In each of the following Synthesis Examples 1 and 2, a polymer having a structure represented by Formula (1) below was synthesized:

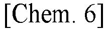

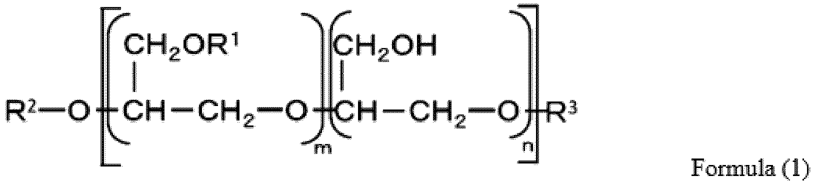

Note that, in Formula (1), the portion within [] is a random copolymer.

### <Synthesis Example 1>

### Synthesis Example 1: Polymer 1, where R¹ = CH₂CH₃; R² = H; R³ = CH₃; m = 4.0, and n = 2.0

64 g of methanol and, as a catalyst, 3.3 g of potassium hydroxide were placed inside an autoclave, air inside the autoclave was replaced with dry nitrogen, and the catalyst was completely dissolved at 80°C while stirring. Next, a mixture of 817 g of ethyl glycidyl ether and 296 g of glycidol was added dropwise over 30 h with a dropping apparatus, followed by stirring for 5 h. The reaction composition was taken out from the autoclave, neutralized with hydrochloric acid to a pH of 6 to 7, and treated under reduced pressure of -0.095 MPa (50 mmHg) at 100°C for 1 h to remove contained moisture. After the treatment, filtration was further carried out to remove any salt generated, and 1120 g of Polymer 1 was obtained.

Various physical property values for the obtained Polymer 1 were determined below.

### (Number average molecular weight)

The number average molecular weight of the obtained Polymer 1 was calculated by gel permeation chromatography (GPC) measurement. Using a SHODEX^{™} GPC101 dedicated GPC system as the system, a SHODEX RI-71s as the differential refractometer, a SHODEX KF-G as the guard column, and three HODEX KF804L installed in series as columns, tetrahydrofuran as the developing solvent was flowed at a flow rate of 1 ml/min at a column temperature of 40°C. 0.1 ml of a 0.1 wt% tetrahydrofuran solution of the resulting reaction product was injected, and a chromatogram represented by refractive index intensity and elution time was obtained using a BORWIN GPC calculation program. A number average molecular weight was determined from this chromatogram using polyethylene glycol as the standard, and was 650.

### (IOB value)

The IOB value of the obtained Polymer 1 was determined as follows, and was 1.04.

More specifically, in Polymer 1, organic values were calculated with carbon at 20 and iso-branch at -10. Inorganic values were calculated with hydroxyl group at 100 and ether bond at 20.
• Methanol site: (1 carbon atom, 1 hydroxyl group) × 1
   Organic value: 20
   Inorganic value: 100
• Glycidol site: (3 carbon atoms, 1 iso-branch, 1 hydroxyl group, 1 ether bond) × 2
   Organic value: (60 - 10) × 2 = 100
   Inorganic value: (100 + 20) × 2 = 240
• Ethyl glycidyl ether site: (5 carbon atoms, 1 iso-branch, 2 ether bonds) × 4
   Organic value: (100 - 10) × 4 = 360
   Inorganic value: 20 × 2 × 4 = 160

From the above, the total organic value is 480 and the total inorganic value is 500, and thus the IOB value is 1.04.

### (Clouding point)

A 5 wt% aqueous solution of the obtained Polymer 1 was prepared and heated to 85°C. The temperature at which a transparent solution was formed was 57°C. Thus, it was found that the clouding point of Polymer 1 was 57°C.

### (Solubility in water)

Solubility in water of the obtained Polymer 1 was determined by the following method, and was 50% by mass or greater.

Specifically, Polymer 1 was mixed with water at a certain concentration, stirred, and then left to stand for one day, followed by determining whether or not there was compatibility by visual observation. In the case of Polymer 1, it was confirmed that there was compatibility with water at a concentration of 50% by mass.

### (Solubility in olive oil)

Solubility in olive oil of the obtained Polymer 1 was determined as follows, and was 1.0% by mass.

Specifically, Polymer 1 was mixed with olive oil at a concentration of 1.0% by mass, stirred, and then left to stand for one day. Appearance was then confirmed by visual observation. "Yes" was indicated if the solution was uniform. In this case, the solubility in olive oil of Polymer 1 was 1.0% by mass.

Various physical property values of Polymer 1 measured above are shown in Table 1 below.

### <Synthesis Example 2>

### Synthesis Example 2: Polymer 2, where R¹ = CH₂CH₃; R² = H; R³ = CH₃; m = 10; and n = 5.0

32 g of methanol and, as a catalyst, 3.3 g of potassium hydroxide were placed inside an autoclave, air inside the autoclave was replaced with dry nitrogen, and the catalyst was completely dissolved at 80°C while stirring. Next, a mixture of 1021 g of ethyl glycidyl ether and 370 g of glycidol was added dropwise over 30 h with a dropping apparatus, followed by stirring for 5 h. The reaction composition was taken out from the autoclave, neutralized with hydrochloric acid to a pH of 6 to 7, and treated under reduced pressure of -0.095 MPa (50 mmHg) at 100°C for 1 h to remove contained moisture. After the treatment, filtration was further carried out to remove any salt generated, and 1351 g of Polymer 2 was obtained.

In the same manner as the case of Polymer 1 mentioned above, various physical property values for the obtained Polymer 2 were determined, and each of the results is shown in Table 1 below.

### [Table 1]

**(Table 1)**

| Polymer | 1 | 2 |
|---|---|---|
| Structure | 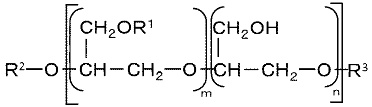 Note that, the portion within [] is a random copolymer. | |
| R¹ | CH₂CH₃ | CH₂CH₃ |
| R² | H | H |
| R³ | CH3 | CH3 |
| Average number of moles added (m, n) | m = 4.0 mol | m = 10 mol |
| | n = 2.0 mol | n = 5.0 mol |
| Number average molecular weight (Mn) | 650 | 1400 |
| IOB value | 1.04 | 0.94 |
| Clouding point (5% aq.) | 57°C | 44°C |
| Solubility (water) | 50% by mass or greater | 50% by mass or greater |
| Solubility (olive oil) *1 | Yes | Yes |

| | | |
|---|---|---|
| *1 Synthesized polymer was mixed with olive oil so as to have a concentration of 1.0 % by mass, and appearance was confirmed. "Yes" was indicated if solution was uniform. | | |

### <<Examples 1 and 2 and Comparative Example 1>>

In Examples 1 and 2, the cosmetic bases 1 and 2 composed of the above Polymers 1 and 2 were used to prepare toners. Feeling of use and moisturizing effect of each toner were examined as follows. Each of the results is shown in Table 3.

In Comparative Example 1, feeling of use and moisturizing effect were examined in the same manner as in the Examples mentioned above, except that the amount of glycerin instead of the polymer was increased.

### <Feeling of use evaluation>

Each toner prepared above, as an evaluation of feeling of use thereof, was comprehensively evaluated by "moisturized feeling", "sticky feeling", "ease of blending into and spreading on skin", and "slimy feeling". Note that, the evaluation was carried out by applying each toner to the skin and by two expert panelists according to the criteria of Table 2 below.

**[Table 2]**

| (Table 2 Evaluation criteria for feeling of use) | | | |
|---|---|---|---|
| Feeling of use | Evaluation criteria | | |
| | A | B | C |
| Moisturized feeling | There is sufficient moisturized feeling | There is some moisturized feeling | Moisturized feeling is noticeably low |
| Sticky feeling | No feeling at all | Slight feeling but not uncomfortable | There is a noticeably sticky and uncomfortable feeling |
| Ease of blending into and spreading on skin | Blends in well and spreads sufficiently | Blends in well but spreads insufficiently | Does not blend in well and spreads insufficiently |
| Slimy feeling | No feeling at all | Slight feeling but not uncomfortable | There is a noticeably slimy and uncomfortable feeling |

### <Moisturizing effect evaluation>

Each toner prepared above, as an evaluation of moisturizing effect thereof, was applied uniformly to the skin, and the moisturizing feel thereafter on the skin was evaluated by two expert panelists. Note that, the evaluation rankings are as follows:
"A": a high moisturizing effect was felt;
"B": some moisturizing effect was felt; and
"C": a moisturizing effect was felt but somewhat insufficient.

### [Table 3]

**(Table 3)**

| Constituent component (% by mass) | | Example 1 | Example 2 | Comparative Example 1 |
|---|---|---|---|---|
| Ethanol | | 3.0 | 3.0 | 3.0 |
| Glycerin | | 5.0 | 5.0 | 7.0 |
| Dipropylene glycol | | 8.0 | 8.0 | 8.0 |
| PEG-8 | | 1.0 | 1.0 | 1.0 |
| Polymer 1 of Synthesis Example 1 | | 2.0 | - | - |
| Polymer 2 of Synthesis Example 2 | | - | 2 | - |
| PPG-13-decyltetradeceth-24 | | 0.2 | 0.2 | 0.2 |
| Citric acid | | 0.01 | 0.01 | 0.01 |
| Na citrate | | 0.09 | 0.09 | 0.09 |
| EDTA-2Na | | 0.03 | 0.03 | 0.03 |
| Phenoxyethanol | | 0.5 | 0.5 | 0.5 |
| Water | | Balance | Balance | Balance |
| Total (% by mass) | | 100 | 100 | 100 |
| Feeling of use evaluation | Moisturized feeling | A | A | B |
| | Sticky feeling | A | A | C |
| | Ease of blending into and spreading on skin | A | A | C |
| | Slimy feeling | A | A | B |
| Moisturizing effect evaluation | | A | A | B |

As is clear from the results in Table 3, the toners of Examples 1 and 2 were all found to have excellent feeling of use and moisturizing effect. In contrast, the toner of Comparative Example 1 was found to have inferior feeling of use and moisturizing effect.

## Claims

1. A cosmetic base, comprising or composed of a polymer having a structure represented by Formula (1) below: 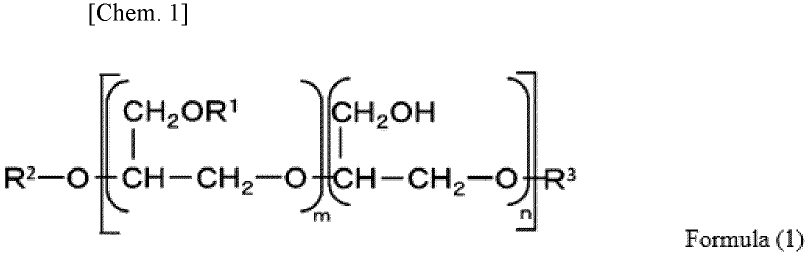
wherein in the Formula (1), R¹ is an alkyl group having 1 to 4 carbon atoms; R² and R³ are each independently a hydrogen atom or an alkyl group having 1 to 4 carbon atoms; and m and n are each independently 1 to 50,
wherein a number average molecular weight of the polymer is 10,000 or less, and
wherein an IOB value of the polymer is 0.4 to 1.8.

2. The cosmetic base according to claim 1, wherein the polymer is a random copolymer.

3. The cosmetic base according to claim 1 or 2, wherein in the Formula (1), R¹ is a methyl group or an ethyl group.

4. The cosmetic base according to claim 1 or 2, wherein in the Formula (1), at least one of R² and R³ is a methyl group.

5. The cosmetic base according to claim 1 or 2, which is used for moisturizing.

6. A cosmetic, comprising the cosmetic base according to claim 1 or 2.
